# EUROPEAN PATENT APPLICATION

(11) **EP 3 343 268 A1**
(43) Date of publication of application: **04.07.2018**
(21) Application number: 16896009.4
(22) Date of filing: 31.03.2016
(51) Int. Cl.: G02B 27/01, A61H 9/00

(54) **GAS BLINDER AND WEARABLE DEVICE**

(71) Applicant: Shenzhen Royole Technologies Co., Ltd., Shenzhen, Guangdong 518052 (CN)
(72) Inventor: LIU, Chang, Shenzhen Guangdong 518052 (CN); YANG, Jinhui, Shenzhen Guangdong 518052 (CN)
(74) Representative: Schwarz & Partner Patentanwälte OG
(86) International application number: PCT/CN2016/078189
(87) International publication number: WO 2017/166229

(57) **Abstract**

A gas blinder (10) comprises a fixed gasbag (11), a gas charging control device (13), and a gas discharging control device (15). The gas charging control device (13) and the gas discharging control device (15) are both electrically connected to a control device (16). The fixed gasbag (11) comprises a first gas intake opening (1101) and a first gas discharging opening (1103). The gas charging control device (13) is in sealed connection with the first gas intake opening (1101), and is used for charging, under the control of the control device (16), gas into the fixed gasbag (11) through the first gas intake opening (1101). The gas discharging control device (15) is in sealed connection with the first gas discharging opening (1103), and is used for discharging, under of the control of the control device (16), gas in the fixed gasbag (11) through the first gas discharging opening (1103). A wearable device using the gas blinder (10). The gas blinder (10) has a good light avoiding effect and a good wear comfort.

## Description

### FIELD

The present disclosure relates to a field of a wearable apparatus, and especially to an air eye cover and a wearable apparatus adopting the air eye cover.

### BACKGROUND

Virtual Reality (VR) refers to producing a virtual world of a three-dimensional space through a computer simulation, providing sensorial simulations such as visual simulation, auditory simulation or tactile simulation for a user, and hence allowing the user to observe objects in the three-dimensional space just like being personally on the scene. In recent years, along with continuous developments of VR technology, distinctive head-mounted display devices are progressively rolled out by a plurality of VR related companies. In order to improve realness of the sensorial simulation, the head-mounted display devices configured to present the virtual reality scene is required to have a great light-blocking effect. Meanwhile, as the user needs to wear the head-mounted display device for a long time when experiencing VR films or games by the head-mounted display devices, a great comfort in the wearing process should be taken in consideration, so as to improve the user experience further. At present, among the current head-mounted display devices, an eye cover contacting face is mostly made of rubber or cloth filled with elastic materials such as sponge. However, as facial features of the users are not totally same, a surface of the eye cover part of the current head-mounted display device cannot be changed flexibly in shape according to a change of the facial feature of the user, thus causing a bad light-blocking effect of the head-mounted display device, and hence influencing a presentation effect of VR films or games. Meanwhile, as the user needs to wear the head-mounted display device all along in the processes of experiencing VR films or games, how to improve the comfort of the head-mounted display device is also a problem to be solved.

### SUMMARY

In consideration of the above-mentioned problems existing in the related art, embodiments of the present disclosure provide an air eye cover, so as to improve a light-blocking effect and a wearing comfort and hence to promote a user experience.

In addition, embodiments of the present disclosure further provide a wearable apparatus adopting the above air eye cover.

The air eye cover includes a fixed gasbag, an air-filling control device and an air-exhausting control device. The air-filling control device and the air-exhausting control device both electrically connect to the control device. The fixed gasbag includes a first air inlet and a first air outlet. The air-filling control device hermetically connects to the first air inlet and is configured to fill air into the fixed gasbag through the first air inlet under control of the control device. The air-exhausting control device hermetically connects to the first air outlet and is configured to exhaust the air in the fixed gasbag through the first air outlet under control of the control device.

The wearable apparatus includes a head-mounted display device and an air eye cover. The air eye cover includes a fixed gasbag, an air-filling control device and an air-exhausting control device. The air-filling control device and the air-exhausting control device both electrically connect to the control device. The fixed gasbag includes a first air inlet and a first air outlet. The air-filling control device hermetically connects to the first air inlet and is configured to fill air into the fixed gasbag through the first air inlet under control of the control device. The air-exhausting control device hermetically connects to the first air outlet and is configured to exhaust the air in the fixed gasbag through the first air outlet under control of the control device. The head-mounted display device includes the control device and a connecting interface. The air eye cover electrically connects to the control device via the connecting interface.

In the air eye cover, the fixed gasbag is provided, and air is filled into the fixed gasbag by the air-filling control device when it is needed to wear the air eye cover so as to fix the air eye cover and fit the air eye cover with a human face via the fixed gasbag. Moreover, the air in the fixed gasbag may be exhausted by the air-exhausting control device in a using process so as to adjust the air pressure in the fixed gasbag, thus realizing suitability and fit for faces of different users. Therefore, a great light-blocking effect and a wearing comfort are provided, thus facilitating improvements of the user experience of the wearable apparatus.

### BRIEF DESCRIPTION OF THE DRAWINGS

In order to specify the technical solutions in embodiments of the present disclosure more clearly, the accompanying drawings required for descriptions of the embodiments in the present disclosure will be simply introduced in the following.
Fig. 1 is a first schematic view of an air eye cover provided in an embodiment of the present disclosure.
Fig. 2 is a schematic view of an air-filling control device and an air-exhausting control device of an air eye cover provided in an embodiment of the present disclosure.
Fig. 3 is a second schematic view of an air eye cover provided in an embodiment of the present disclosure.
Fig. 4 is a third schematic view of an air eye cover provided in an embodiment of the present disclosure.
Fig. 5 is a schematic view of a wearable apparatus provided in an embodiment of the present disclosure.

### DETAILED DESCRIPTION

The technical solutions in embodiments of the present disclosure will be described definitely and completely in combination with accompanying drawings of embodiments of the present disclosure. Apparently, the described embodiments are only a part of embodiments of the present disclosure instead of all of the embodiments. All of the other embodiments obtained by those skilled in the art based on the embodiments of the present disclosure without a creative labor belong to a protection scope of the present disclosure.

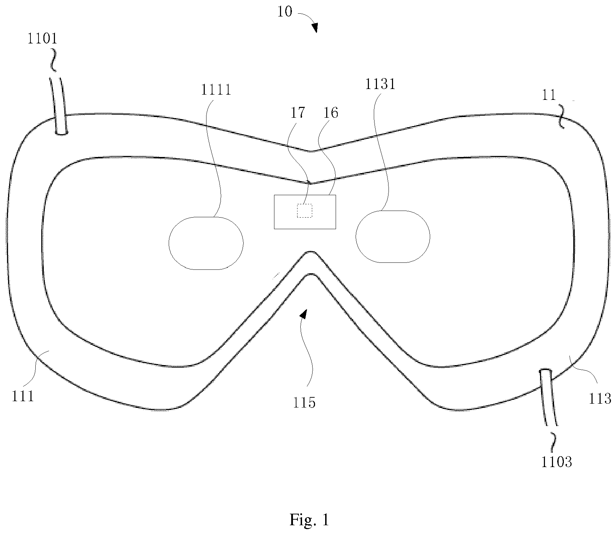

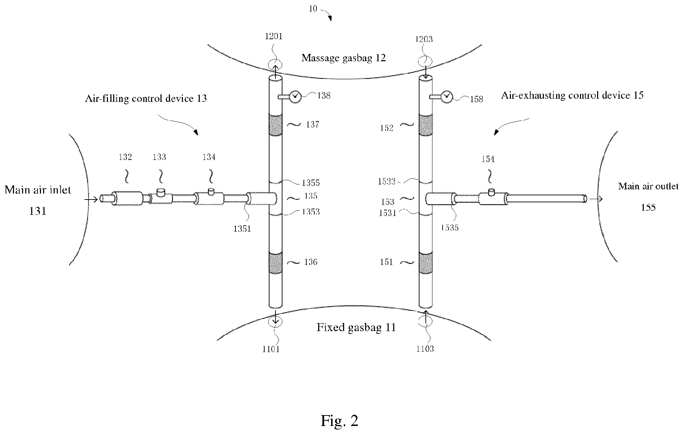

Referring to Fig. 1 and Fig. 2 together, an embodiment of the present disclosure provides an air eye cover 10, including a fixed gasbag 11, an air-filling control device 13, an air-exhausting control device 15 and a control device 16. The air-filling control device 13 and the air-exhausting control device 15 both electrically connect with the control device 16. The control device 16 is a Central Processing Unit (CPU), a Field-Programmable Gate Array (FPGA), a microprocessor, a controller or the like. Specifically, the air eye cover 10 may electrically connect to an electronic apparatus including a control device through a communication interface. The air eye cover may be controlled by the control device of the electronic apparatus, for example a control device of a head-mounted display device. It could be understood that the control device 16 may be incorporated to the air eye cover 10, for example the control device 16 may be a microprocessor arranged in the air eye cover 10, or the control device 16 may also run independently from the air eye cover 10, for example the control device 16 may be the control device of the head-mounted display device. The fixed gasbag 11 includes a first air inlet 1101 and a first air outlet 1103. The air-filling control device 13 hermetically connects to the first air inlet 1101 and is configured to fill air into the fixed gasbag 11 through the first air inlet 1101 under a control of the control device 16. The air-exhausting control device 15 hermetically connects to the first air outlet 1103 and is configured to exhaust the air in the fixed gasbag 11 through the first air outlet 1103 under the control of the control device 16.

In an embodiment, the air-filling control device 13 includes a main air inlet 131, an air pump 132 and an air-filling solenoid valve 134. The air pump 132 includes a first end connecting to the main air inlet 131 and a second end hermetically connecting to the first air inlet 1101 via the air-filling solenoid valve 134. The air-exhausting control device 15 includes an air-exhausting solenoid valve 154 and a main air outlet 155. The air-exhausting solenoid valve 154 has a first end hermetically connecting to the first air outlet 1103 and a second end hermetically connecting to the main air outlet 155.

The fixed gasbag 11 includes a first fixed portion 111 and a second fixed portion 113. The first fixed portion 111 is provided with a first through hole 1111, the second fixed portion 113 is provided with a second through hole 1131, and a first reversed V-shaped structure 115 is formed at a position where the first fixed portion 111 and the second fixed portion 113 connect to each other. When the fixed gasbag 11 is filled with air and closely fits with human face, the first reversed V-shaped structure 115 is perched on a bridge of a nose, and the first through hole 1111 and the second through hole 1131 are corresponding to positions of human eyes respectively. It could be understood that the first fixed portion 111 and the second fixed portion 113 connect to each other inside the gasbag, that is, the air may flow freely between the first fixed portion 111 and the second fixed portion 113. The first air inlet 1101 and the first air outlet 1103 may be provided at any positions in the fixed gasbag 11. In the present embodiment, the first air inlet 1101 is provided in the first fixed portion 111, and the first air outlet 1103 is provided in the second fixed portion 113.

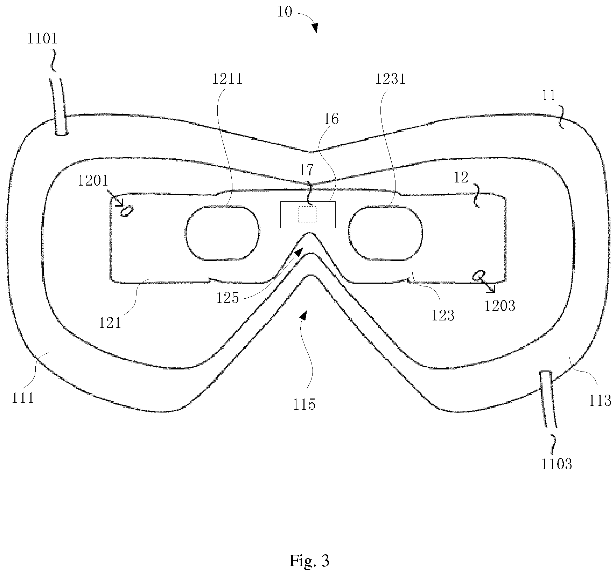

Referring to Fig. 2 and Fig. 3 together, the air eye cover 10 further includes a massage gasbag 12. The massage gasbag 12 includes a second air inlet 1201 and a second air outlet 1203. The air-filling control device 13 also hermetically connects to the second air inlet 1201 and is configured to fill air into the massage gasbag 12 through the second air inlet 1201 under the control of the control device 16. The air-exhausting control device 15 also hermetically connects to the second air outlet 1203 and is configured to exhaust the air in the massage gasbag 12 through the second air outlet 1203 under the control of the control device 16.

The massage gasbag 12 includes a first massage portion 121 and a second massage portion 123. The first massage portion 121 is provided with a first via hole 1211, the second massage portion 123 is provided with a second via hole 1231. A second reversed V-shaped structure 125 is formed at a position where the first massage portion 121 and the second massage portion 123 connect to each other. When the massage gasbag 12 is embedded in the fixed gasbag 11, the first via hole 1211 aligns with the first through hole 1111, the second via hole 1231 aligns with the second through hole 1131, and the second reversed V-shaped structure 125 perches on the first reversed V-shaped structure 115. It could be understood that, the massage gasbag 12 may be embedded in the fixed gasbag 11 completely and penetrate the fixed gasbag 11, or partially embedded in the fixed gasbag 11 at a side of the fixed gasbag 11 facing the human face. When the massage gasbag 12 is completely embedded in the fixed gasbag 11 and penetrates the fixed gasbag 11, a through hole (not illustrated in the drawings) corresponding to an outline of the massage gasbag 12 needs to be reserved in the fixed gasbag 11. The massage gasbag 12 is arranged in the through hole and exposed out of the side of the fixed gasbag 11 facing the human face and a side thereof away from the human face. It could be understood that, in the solution the massage gasbag 12 is completely embedded in the fixed gasbag 11 and penetrates the fixed gasbag 11, it is only needed to define the through hole in the fixed gasbag 11, but not to define the first through hole 1111 and the second through hole 1131.

A detachable embedding manner may be provided in the above-mentioned embedding manners, so as to provide a free choice for the user. In other embodiments, the massage gasbag 12 and the fixed gasbag 11 may be channels, where the air flows, separately provided inside the eye cover, that is, channels of the two gasbags are independent from each other. It could be understood that, the accompanying drawings only serve as principle views and are not intended for limiting forms of the air eye cover 10, the massage gasbag 12 and the fixed gasbag 11. Based on such principle, the form of the eye cover as well as the form and position of the specific channel of the gasbag can be designed flexibly by those skilled in the related art according to design requirements of product.

Referring to Fig. 2 again, the air-filling control device 13 includes the main air inlet 131, the air pump 132, the air-filling solenoid valve 134, an air-filling switch valve 135, a first air-filling check valve 136 and a second air-filling check valve 137. The air-filling switch valve 135 includes an input end 1351, a first output end 1353 and a second output end 1355. The first end of the air pump 132 connects to the main air inlet 131, and the second end of the air pump 132 hermetically connects to the input end 1351 via an air release valve 133 and the air-filling solenoid valve 134 in order. The first output end 1353 hermetically connects to the first air inlet 1101 via the first air-filling check valve 136. The second output end 1355 hermetically connects to the second air inlet 1201 via the second air-filling check valve 137.

It could be understood that, the air pump 132, the air-filling solenoid valve 134 and the air-filling switch valve 135 all electrically connect to the control device 16. When the control device 16 controls the air pump 132 to switch on so as to suck in air and the air-filling solenoid valve 134 is opened, the air enters the fixed gasbag 11 or the massage gasbag 12. When the control device 16 controls the air-filling switch valve 135 to switch to the first output end 1353 so as to output air thereat, the air enters the fixed gasbag 11 from the first output end 1353 through the first air-filling check valve 136 and the first air inlet 1101. When the control device 16 controls the air-filling switch valve 135 to switch to the second output end 1355 so as to output air thereat, the air enters the massage gasbag 12 from the second output end 1355 through the second air-filling check valve 137 and the second air inlet 1201.

Specifically, the air-filling control device 13 may further include the air release valve 133 provided between the air pump 132 and the air-filling solenoid valve 134 and configured to release air when an air input volume is too large. The air release valve 133 may be a mechanical valve or an electronic control valve. When the air release valve 133 is a mechanical valve, if a pressure in a pipe is larger than an opening pressure value of the mechanical valve, the air release valve 133 is opened to release the air. When the air is released such that the pressure value recovers to a standard value, that is the pressure value is less than the opening pressure value, the air release valve 133 comes into a closed state. The air release valve 133 is used as a protection measure when a too large amount of air exists between the air pump 132 and the air-filling solenoid valve 134.

The air-exhausting control device 15 includes a first air-exhausting check valve 151, a second air-exhausting check valve 152, an air-exhausting switch valve 153, an air-exhausting solenoid valve 154 and a main air outlet 155. The air-exhausting switch valve 153 includes a first input end 1531, a second input end 1533 and an output end 1535. The first input end 1531 hermetically connects to the first air outlet 1103 via the first air-exhausting check valve 151. The second input end 1533 hermetically connects to the second air outlet 1203 via the second air-exhausting check valve 152. The input end 1535 hermetically connects to the main air outlet 155 via the air-exhausting solenoid valve 154.

It could be understood that the air-exhausting switch valve 153 and the air-exhausting solenoid valve 154 both electrically connect to the control device 16. When the control device 16 controls the air-exhausting solenoid valve 154 to open, the air in the fixed gasbag 11 or the massage gasbag 12 can be exhausted through the main air outlet 155. When the control device 16 controls the air-exhausting switch valve 153 to switch to the first input end 1531 so as to input air thereat, the air in the fixed gasbag 11 is exhausted out of the main air outlet 155 through the first air-exhausting check valve 151, the first input end 1531, the output end 1535 and the air-exhausting solenoid valve 154. When the control device 16 controls the air-exhausting switch valve 153 to switch to the second input end 1533 so as to input air thereat, the air in the massage gasbag 12 is exhausted out of the main air outlet 155 through the second air-exhausting check valve 152, the second input end 1533, the output end 1535 and the air-exhausting solenoid valve 154. In the present embodiment, the air release valve 133, the air-filling solenoid valve 134 and the air-exhausting solenoid valve 154 are normally-open elements when de-energized, that is, the de-energization of the valve corresponds to the open of the valve, and the energization of the valve corresponds to the close of the valve.

The air-filling control device 13 further includes a first flowmeter 138. The first flowmeter 138 is disposed between the second air-filling check valve 137 and the second air inlet 1201 and configured to detect an air-intake flow rate of the massage gasbag 12. The air-exhausting control device 15 further includes a second flowmeter 158. The second flowmeter 158 is disposed between the second air-exhausting check valve 152 and the second air outlet 1203 and configured to detect an air-exhaust flow rate of the massage gasbag 12. In the present embodiment, the air-intake flow rate and the air-exhaust flow rate are controlled by a conjugate square wave control signal produced by the control device 16, a massage frequency of the massage gasbag 12 is decided by a duty ratio of the conjugate square wave control signal, and a massage strength of the massage gasbag 12 is decided by the air-intake flow rate and the air-exhausting flow rate.

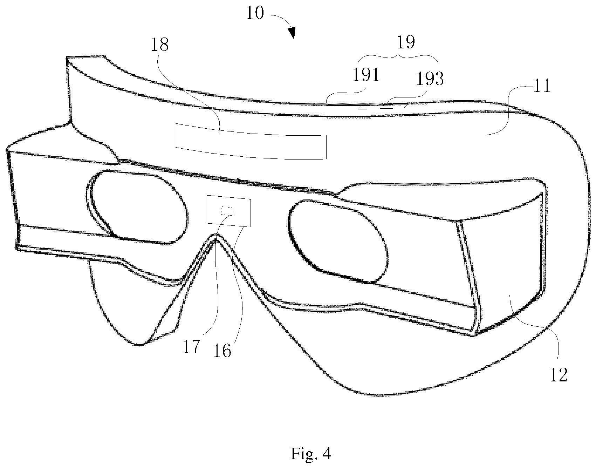

Referring to Fig. 4, the air eye cover 10 further includes a connector 17 disposed to the massage gasbag 12 or the fixed gasbag 11 (as illustrated in Fig. 1). The air-filling control device 13 and the air-exhausting control device 15 electrically connect to the control device 16 via the connector 17 so as to receive control signals for air filling, air exhausting, massage or the like sent from the control device 16. The air eye cover 10 further includes a thin film pressure sensor 18 disposed to the fixed gasbag 11. The thin film pressure sensor 18 electrically connects to the control device 16 and is configured to detect an air pressure in the fixed gasbag 11, and triggers an air filling of the fixed gasbag 11 by the air-filling control device 13 or triggers an air exhaust of the fixed gasbag 11 by the air-exhausting control device 15 according to a change of the air pressure until the air pressure in the fixed gasbag 11 is equal to a preset pressure.

Specifically, in a using process of the air eye cover 10, the thin film pressure sensor 18 detects the air pressure in the fixed gasbag 11 and transmits the detected air pressure to the control device 16. The control device 16 judges whether the air pressure arrives at a preset pressure value. When the air pressure is less than the preset pressure value, the control device 16 controls the air pump 132 to switch on, the air release valve 133 to close, the air-filling solenoid valve 134 to open, and the air-filling switch valve 135 to switch to the first output end 1353 to output air thereat, so as to fill air into the fixed gasbag 11. When the air pressure falls into a certain range of the preset pressure value, the fixed gasbag 11 is stopped from being filled with air. Moreover, when the air pressure in the fixed gasbag 11 is detected to be larger than the preset pressure value, the control device 16 controls the air-exhausting solenoid valve 154 to open and controls the air-exhausting switch valve 153 to switch to the first input end 1531 to input air thereat, so that the air in the fixed gasbag 11 can be exhausted appropriately until the air pressure in the fixed gasbag 11 falls into the certain range of the preset pressure value.

It could be understood that the preset pressure value may be predetermined according to requirements of a degree of tightness when the user herself/himself wears the air eye cover. Specifically, the air-filling control device 13 may include an air-filling mechanical switch, and the air-exhausting control device 15 may include an air-exhausting mechanical switch. When the user uses the air eye cover 10 for the first time, the air filling and the air exhaust of the fixed gasbag 11 may be controlled by means of the air-filling mechanical switch and the air-exhausting mechanical switch, and the air-filling and air-exhausting processes are stopped until the air pressure in the fixed gasbag 11 arrives at an optimum pressure perceived by the user. The control device 16 detects a pressure value of the optimum pressure by means of the thin film pressure sensor 18 and records the pressure value of the optimum pressure as the preset pressure value.

The air eye cover 10 further includes an electric heating device 19. The electric heating device 19 includes a heating film 191 and a thin film temperature sensor 193. The heating film 191 is disposed at a side of the massage gasbag 11 facing the human face, electrically connects to the control device 16 and is configured to transform electric energy provided by the control device 16 into thermal energy. The thin film temperature sensor 193 is disposed at a side of the heating film 191 away from the human face, electrically connects to the control device 16 and is configured to detect a temperature of the heating film. It could be understood that a temperature of the electric heating device 19 can be set by the control device 16. It could be understood that in the air eye cover 10 according to embodiments of the present disclosure, various components where the air flows hermetically connect to one another via flexible guiding pipes.

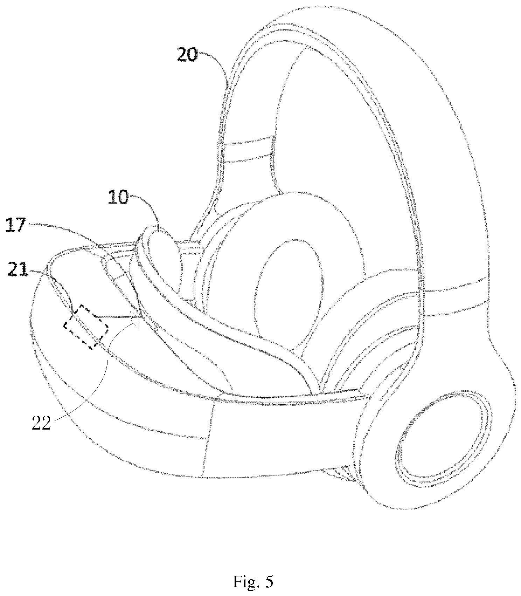

In addition, embodiments of the present disclosure further provide a wearable apparatus including a head-mounted display device 20 and an air eye cover 10, as illustrated in Fig. 5. The head-mounted display device 10 includes a control device 21 and a connecting interface 22. The air eye cover 10 connects to the connecting interface 22 via the connector 17 so as to complete an electrical connection between the air eye cover 10 and the control device 21. As a controller of the air eye cover 10, the control device 21 is used for controlling functions of the air eye cover, such as air filling, air exhausting, heating, massage or the like. For example, the head-mounted display device 20 may display a menu for operating and controlling the air eye cover 10, such that the functions of the air eye cover 10, such as the air filling, the air exhausting, the heating, the massage or the like, may be controlled through the menu. It is possible to refer to related descriptions of the embodiments illustrated in Fig. 1 to Fig. 4 for specific structures and function realizations of the air eye shade 10, which will not be elaborated here.

In the air eye cover, the fixed gasbag is provided, and air is filled into the fixed gasbag by the air-filling control device when it is needed to wear the air eye cover, so as to fix the air eye cover and fit the air eye cover with the human face via the fixed gasbag. Moreover, the air in the fixed gasbag may be exhausted by the air-exhausting control device in the using process, so as to adjust the air pressure in the fixed gasbag, thus realizing suitability and fit for faces of different users. Therefore, a great light-blocking effect and a wearing comfort are provided, thus facilitating improvements of the user experience of the wearable apparatus. Also, the air eye cover may directly adopt atmosphere as an air source and hence have characteristics of environmental friend and light weight.

The disclosures above are only better embodiments of the present disclosure and cannot be used to limit a claim scope of the present disclosure. All or a part of the processes for realizing the above embodiments can be understood by those ordinarily skilled in the art. The equivalent changes made based on the above embodiments still belong to a scope contained by the present disclosure.

## Claims

1. An air eye cover, comprising a fixed gasbag, an air-filling control device and an air-exhausting control device, the air-filling control device and the air-exhausting control device both electrically connect to a control device; wherein the fixed gasbag comprises a first air inlet and a first air outlet, the air-filling control device hermetically connects to the first air inlet and is configured to fill air into the fixed gasbag through the first air inlet by control of the control device, and the air-exhausting control device hermetically connects to the first air outlet and is configured to exhaust the air in the fixed gasbag through the first air outlet by the control of the control device.

2. The air eye cover according to claim 1, wherein the air-filling control device comprises a main air inlet, an air pump and an air-filling solenoid valve, a first end of the air pump connects to the main air inlet and a second end of the air pump hermetically connects to the first air inlet via the air-filling solenoid valve, and the air-exhausting control device comprises an air-exhausting solenoid valve and a main air outlet, a first end of the air-exhausting solenoid valve hermetically connects to the first air outlet and a second end of the air-exhausting solenoid valve hermetically connects to the main air outlet.

3. The air eye cover according to claim 2, further comprising an air release valve connecting the air pump and the air-filling solenoid valve and configured to release air when a volume of the air between the air pump and the air-filling solenoid valve is larger than a preset standard.

4. The air eye cover according to claim 2, further comprising a massage gasbag, wherein the massage gasbag comprises a second air inlet and a second air outlet, the air-filling control device also hermetically connects to the second air inlet and is configured to fill air into the massage gasbag through the second air inlet under the control of the control device, the air-exhausting control device also hermetically connects to the second air outlet and is configured to exhaust the air in the massage gasbag through the second air outlet under the control of the control device.

5. The air eye cover according to claim 4, wherein the air-filling control device further comprises an air-filling switch valve, a first air-filling check valve and a second air-filling check valve, the air-filling switch valve comprises an input end, a first output end and a second output end; the air-filling solenoid valve hermetically connects to the first air inlet via the input end, the first output end and the first air-filling check valve in order, and the air-filling solenoid valve hermetically connects to the second air inlet via the input end, the second output end and the second air-filling check valve in order.

6. The air eye cover according to claim 5, wherein the air-exhausting control device further comprises a first air-exhausting check valve, a second air-exhausting check valve and an air-exhausting switch valve; the air-exhausting switch valve comprises a first input end, a second input end and an output end; the first input end hermetically connects to the first air outlet via the first air-exhausting check valve, the second input end hermetically connects to the second air outlet via the second air-exhausting check valve, and the input end hermetically connects to the main air outlet via the air-exhausting solenoid valve.

7. The air eye cover according to claim 1, further comprising a thin film pressure sensor disposed on the fixed gasbag, wherein the thin film pressure sensor electrically connects to the control device and is configured to detect an air pressure in the fixed gasbag, the control device is configured to trigger an air filling of the fixed gasbag by the air-filling control device or trigger an air exhaust of the fixed gasbag by the air-exhausting control device according to a change of the air pressure change until the air pressure in the fixed gasbag is equal to a preset pressure.

8. The air eye cover according to claim 6, wherein the air-filling control device further comprises a first flowmeter, and the first flowmeter is disposed between the second air-filling check valve and the second air inlet and configured to detect an air-intake flow rate of the massage gasbag; and
the air-exhausting control device further comprises a second flowmeter, and the second flowmeter is disposed between the second air-exhausting check valve and the second air outlet and configured to detect an air-exhausting flow rate of the massage gasbag.

9. The air eye cover according to claim 8, wherein the air-intake flow rate and the air-exhausting flow rate are controlled by a conjugate square wave control signal produced by the control device, a massage frequency of the massage gasbag is decided by a duty ratio of the conjugate square wave control signal, and a massage strength of the massage gasbag is decided by the air-intake flow rate and the air-exhausting flow rate.

10. The air eye cover according to claim 1, further comprising an electric heating device, wherein the electric heating device comprises a heating film and a thin film temperature sensor, the heating film is disposed at a side of the massage gasbag facing human face, electrically connects to the control device and is configured to transform electric energy provided by the control device to thermal energy, the thin film temperature sensor is disposed at a side of the heating film away from the human face, electrically connects to the control device and is configured to detect a temperature of the heating film.

11. The air eye cover according to claim 1, further comprising a connector, wherein the air-filling control device and the air-exhausting control device electrically connect to the control device via the connector.

12. A wearable apparatus, comprising a head-mounted display device and an air eye cover according to any one of claims 1 to 11, wherein the head-mounted display device comprises a control device and a connecting interface, and the air eye cover electrically connects with the control device via the connecting interface.
